# NEW EUROPEAN PATENT SPECIFICATION

(11) **EP 0 785 714 B2**
(45) Date of publication and mention of the opposition decision: **21.05.2003**
(45) Mention of the grant of the patent: 30.08.2000
(21) Application number: 95934953.1
(22) Date of filing: 13.10.1995
(51) Int. Cl.: A01N 37/02, A01N 37/04, A01N 37/06, A01N 37/34, A01N 31/02, A61K 31/19, A61K 31/045, A61K 7/48, B27K 3/50, C11D 7/26, C11D 7/60

(54) **ANTIMICROBIAL COMPOSITION**
ANTIMIKROBIELLE ZUSAMMENSETZUNG
COMPOSITION ANTIMICROBIENNE

(30) Priority: 14.10.1994 SE 9403541
(43) Date of publication of application: 30.07.1997
(73) Proprietor: Moberg, Sven, 433 02 Partille (SE)
(72) Inventor: Moberg, Sven, 433 02 Partille (SE)
(74) Representative: Bergvall-Eftring, Stina Lena
(86) International application number: SE9501191
(87) International publication number: WO96011572

(56) References cited:
- EP-A- 0 033 111
- EP-A- 0 241 779
- EP-A- 0 582 360
- WO-A-87/04617
- WO-A-89/00853
- WO-A-94/09755
- DE-A- 3 042 507
- DE-A- 3 227 126
- DE-C- 4 311 713
- GB-A- 1 388 836
- GB-A- 1 555 796
- SE-B- 464 060
- ACTA DERMATO-VENEREOLOGICA, Volume 71, 1991, TUULA KINNUNEN et al., "Antibacterial and Antifungal Properties of Propylene Glycol, Hexylene Glycol and 1,3 Butylene Glycol in Vitro", pages 148-150.
- ACTA DERMATOVENER, Volume 60, 1980, JAN FAERGEMANN et al., "Propylene Glycol in the Treatment of Tinea Versicolor", pages 92-93.
- MARTINDALE W., "The Extra Pharmacopoeia", 1977, Twenty-seventh Edition, (London), pages 212, 213, 651, 652, 738-744, 1275-1276.
- FOOD CHEMISTRY, Volume 4, No. 4, 1979, G. POLI et al., "Virucidal Activity of Organic Acids", pages 251-257.
- STN INTERNATIONAL, File CA, Chemical Abstracts, Volume 113, No. 2, 9 July 1990, (Columbus, Ohio, US), LION HYGIENE K.K., "Detergent Compositions for Cleaning Hot Griddles During Use", Abstract No. 8476; & JP,A,02 047 200, (16-02-90), Heisei.
- STN INTERNATIONAL, File CA, Chemical Abstracts, Volume 108, No. 8, 22 February 1988, (Columbus, Ohio, US), ALL INDIA INSTITUTE OF MEDICAL SCIENCES, "Pharmaceutical Containing Lactic Acid and Glycerin and a Detergent for the Treatment of Acne", Abstract No. 62508; & IN,A,157 171, (01-02-86).

## Description

The invention relates to the use of a composition having antimicrobial and hygroscopic properties comprising carboxylic acids or salts thereof, having up to 10 carbon atoms and C₃-C₁₀-diols as a mixture for cleansing, disinfection, surface treatment, impregnation and for antimicrobial treatment.

### Background of the invention

From T. Kinnunen at al., Acta Derm. Venereol (Stockholm 1991) vol. 71:148-150 it is known that diols (glycols) have antimicrobial effect against both fungi and bacteria in vitro. According to Kinnunen 10-30 % hexylene glycol showed antimicrobial effect within 20 hours, 5 % hexylene glycol a certain antimicrobial effect, while 1 % of the agent lacked effect. On comparison 30 % butylene glycol and 30 % propylene glycol were about as effective as 10 % hexylene glycol. Diols occur as solvents in various dermatological vehicles since then they are atoxic to skin epithelium. Propylene glycol is a commonly occurring solvent in dermatolological vehicles. However, also diols having longer carbon chains, such as, for example, butylene glycol and hexylene glycol have recently started to be used in vehicles instead of propylene glycol, mainly due to their stronger antimicrobial properties.

Furthermore, diols may be used as antifreeze agents, as is well known.

Furthermore, propylene glycol in a 50 % aqueous solution was shown to be effective against one skin disease, Pityriasis versicolor, caused by the yeast fungi Pityrosporum ovale (cf. Jan Faergemann, Acta Dermatovener (Stockholm) 60:92-93, 1980). Robertson et al., has in J. Infect. Dis. 83:124-137, 1948, examined the bactericidal effect of i. a. glycol vapours and lactic acid on airborne organisms and illustrates also in his study that lactic acid is the most air-sterilizing of the tested compounds. It is, however, pointed out on page 136, left column, that lactic acid is not a suitable substance. Neither is there in any of the references any information which would lead a person skilled in the art to draw the conclusion that a combination of carboxylic acid having up to 10 carbon atoms and C₃-C₁₀-diols has a synergistic acting antimicrobial effect on both fungi organisms and bacteria.

In Current Therapeutic Research (43:547-551, 1988) Faergemann compares the antimicrobial effect of two diols, propane-1,2-diol and 2-methyl-2,4-pentanediol, and describes the advantages of these diols when added to a skin cream. However, nothing is mentioned about a combination of carboxylic acids or that such a combination could give even lower MIC-values and improved treatment results. Despite the statement that these two diols may serve as a preservative it is stated on page 550 that alclometasone dipropionate cream and Essex cream contain a preservative, i.e. chlorocresol, which is known to cause allergic reactions.

The Swedish patent No. 464 060 relates to the use of pentane diol or hexane diol for preparing an agent for treating infections caused by the herpes virus.

The Swedish patent application No. 8802257-9 relates to the use of a composition containing aluminium acetotartrate (alsol solution) and a diol having antimicrobial properties for preparing an agent aimed at reducing skin irritation, particularly in the nappy region. Furthermore, it has been reported that the killing of microorganisms is potentialized by the combination propylene glycol and aluminium acetotartrate.

It is previously known that lactic acid (Acidum lacticum) has hygroscopic and acidifying properties and is included, i.a. in foodstuffs, skin preparations and soap products.

WO 89/00853 relates to a treatment agent containing salicylic acid in combination with aliphatic 1,2-diol and fatty acid esters for local therapy for flaking skin diseases in human and veterinary medicine. Salicylic acid has well known anti-flaking properties.

WO 94/09755 discloses the use of salicylic acid in combination with propylene glycol for the treatment of flaking skin diseases, such as, eczema and psoriasis. However, salicylic acid is not included in the present invention.

It is previously known that certain carboxylic acids can have antiviral properties in vitro against respiratory virus (DE 3227126) as well as herpesvirus, orthomyxovirus and rhabdovirus (G. Poli et al "Virucidal activity of organic acids", Food Chemistry, vol. 4, No. 4, 1979, pages 251-257). It is also known that certain carboxylic acids, for example propionic acid have antimycotic effects and some other carboxylic acids, such as lactic acid have known antibacterial effects (The Extra Pharmacopoeia, W. Martindale, London, 1977, 27th Ed., pages 651-652 and 738-744, 1275-1276.

GB, A1 155 796 discloses the use of alkyl lactate in combination with alkylene glycol as a solvent for the treatment of acne. Certain carboxylic acids are used as buffers to adjust pH and prevent hydrolysis. Also GB, A1 1 388 836 and EP 0 150 914 disclose the use of alkyl lactate in combination with alkylene glycol (propylene glycol for example) as a solvent for the treatment of acne.

According to EP A2 0 241779 organic acids are used to dissolve iron oxide together with sorbitol as a surfactant. In certain cases sorbitol can be replaced by glycols.

According to Chemical Abstracts, Vol. 113 (1990) abst. No. 8476, JP, A2, 0 2047200 warm baking trays are cleaned mechanically with caboxylic acids and glycols.

DE, C1 4311713 discloses the use of citric acid or tartaric acid dissolved in propylene glycol or polyethylene glycol. The composition is used on glass or plexiglass surfaces in shower cabinets where the acid has the role of dissolving deposits of lime and the glycol to give a sufficient viscosity so that the composition stays in place for the time it takes for the acid to work. Carboxylic acids have a similar function, in DE, A1 3042507 for the purpose of cleaning surfaces on enamelled goods. Additives of hygroscopically active diols may also be included.

EP 0 033 111 relates to a combination of organic acid and alcohol to mechanically loosen deposits of lime, oil and fat on the interior surface of dishwashers.

IN-A-157171 discloses agents containing 1-10% lactic acid in propylene glycol as a solvent, for treatment of acne. WO-A-8704617 discloses combinations of urea and propylene glycol for skin treatment. Low levels of lactic acid are used, but purely as acidifying agent.

It is known that many antimicrobial substances presently used may cause side effects such as, for example, allergies, and are therefore unsuitable for use e.g. in skin preparations.

Cleaning agents of today, such as washing-up liquid, soap etc., which are often characterised by a basic pH-value, are easily contaminated and, furthermore, are hardly kind to the skin. Various additives often mean that the cleaning agents are no longer either environmentally kind or kind to the skin and thus are not readily accepted by the consumer.

Neither is there currently a suitable agent for cleaning skin, leather, plastic, metal and wooden surfaces.

The agents of today used for cleaning surfaces of biological material often contain ethyl alcohol and isopropanol, which appear to be desiccating.

Peeling agents sometimes contain abrasives which may cause sores on the skin, which secondarily may act as an approach for bacteria, therefore an effective antimicrobial agent kind to the skin having peeling effect without harmful additives would fulfill a long sought after need.

Another current problem is that impregnation agents and anti-microbial additives to paint are often toxic, highly allergenic and are not environmentally friendly.

The forming of mould is also a great problem in the handling of wood and in waste paper recycling.

The medical care of today lacks also a user-kind agent having effect on microbes on the skin and for the diseases caused by this. Antibiotic additives in skin preparations may cause resistance and allergy problems.

The cited technique relates i.a. to different forms of mechanical cleaning where, for example, deposits of lime and iron oxide are dissolved by carboxylic acids. The present invention relates, however, to an antimicrobial and hygroscopic activating composition. The synergistic active antimicrobial properties of the active components according to the invention in the stated areas of use are, thus, not previously described.

Thus, for a long time there has been a need for an environmentally friendly, low allergenic, kind-to-the-skin and non-dangerous preparation having hygroscopic and antimicrobial acting properties, which may be used for widely different areas such as for cleansing, disinfection, surface treatment and impregnation or for producing biologically decomposable materials.

### Description of the invention

Said problems are solved according to the invention by the use of a composition containing carboxylic acids or salts thereof having up to 10 carbon atoms and C₃-C₁₀-diols which give an increased antimicrobial effect and a wide antimicrobial spectrum having great inhibitory effect on several dermatophytes, yeast fungi, mould fungi, bacteria and virus. The composition is furthermore kind to the skin, environmentally kind, low allergenic, keratolytic active and has also a hygroscopic effect and does not evaporate as fast as e.g. ethanol. From the research carried out by the inventor in developing the invention an increased antimicrobial effect was obtained by a combination of diols and carboxylic acids according to the invention in tests on different microorganisms. Preferably C₃-C₆-diols were used.

Examples of C₃-C₁₀-diols which may be used according to the invention are propylene glycol; butylene glycol; pentanediol, hexylene glycol, heptanediol, octanediol, nonanediol, decanediol.

The term carboxylic acids having up to 10 carbon atoms, which may be used according to the invention, relates to saturated and unsaturated, straight and branched aliphatic mono-, di- and poly-carboxylic acids having up to 10 carbon atoms, araliphatic and aromatic dicarboxylic acids, oxy and hydroxy carboxylic acids having up to 8 carbon atoms. Examples hereof are formic acid, acetic acid, propionic acid, butyric acid, valeric acid, caproic acid, caprylic acid, capric acid, sorbic acid, oxalic acid, malonic acid, fumaric acid, succinic acid, glutaric acid, adipic acid, pimelic acid, oxalacetic acid, phtalic acid, glycolic acid, citric acid, lactic acid, glucuronic acid, glyceric acid, malic acid, tartaric acid, tartronic acid, hydroxibutyric acid, hydroxipropionic acid and pyruvic acid.

The combination which is used according to the invention may be a mixture or a chemical composition in the form of an ester, a polyester or a polymer.

A chemical composition has the advantage over a mixture in that it is relatively stable and is not so easily leached with water, if, for example it is used for the purpose of impregnating. Since the chemical composition is biologically decomposable small amounts of carboxylic acid and diol may slowly be released, whereby the material maintains its antimicrobial effect during a significantly longer time. A bond between carboxylic acids and diols may be made more or less stable so that sufficiently large amounts of the decomposable products diol and carboxylic acid are released for adaptation for various purposes.

A chemical fixing of the carboxylic acids and diols may be achieved with the aid of polyester bonds or longer chains of diol and carboxylic acid together, so called polymers. A polymer of only lactic acid, so called polylactides, are used today for producing "non-woven" material, which, i.a. may be used for nappies, sutures and packing material. For these areas of use the possible antimicrobial effect of a material is of great importance.

A chemical compound of diol and carboxylic acid has similar areas of use, i.e. in the production of nappies, dressings, sanitary products, sutures and packing materials, and also as an additive for wood and paper products.

The composition according to the invention may contain additives such as water, C₁-C₈-alcohols, oils preferably vegetable oils such as peanut oil, olive oil, rape seed oil, linseed oil, tall oil and castor oil with or without being combined with emulsifying agents. Also surfactants may be added to strengthen the cleansing effects. Urea and/or polyethylene glycol may also be included. Other additives may be antimycotics, preferably azole derivatives, allyl amines and amorolfine; antiviral agents, preferable idoxyuridine, acyclovir, phosphonio formic acid, podophyllotoxin; antibacterial agents such as biguanides and amidines, quinolines, benzoyl peroxide, bibrocatole, clindamycin, neomycin, fucidinic acid, mupirocin, sulphur; glucocorticoides, preferably hydrocortisone and flouro substituted steroids, gels and enzymes.

According to the invention a composition is used comprising carboxylic acids or salts thereof, as defined above, and C₃-C₁₀-diols in products for cleansing, disinfection or antimicrobial effect, surface treatment and impregnating or for the production of biologically decomposable material. The invention comprises also mixtures of several carboxylic acids and diols where diols having shorter carbon chains serve as solvents for diols having longer carbon chains.

The included amounts of carboxylic acid when in use according to the invention constitute 0.1-60 percent by weight and C₃-C₁₀-diol constitute 0.1-99.9 percent by weight.

According to a preferred embodiment of the invention 0.1-40 percent by weight carboxylic acid and 0.15-91 percent by weight diol is used.

According to an especially preferred embodiment of the invention 0.12-40 percent by weight acetic acid, citric acid, tartaric acid, lactic acid and 0.38-91 percent by weight propylene glycol, butylene glycol, pentanediol and hexylene glycol is used, wherein one or several carboxylic acids and diols may be included.

The minimal inhibitory concentration (MIC) for propylene glycol (100%) tested on the bacterium Staphylococcus aureus and the dermophyte Tricophyton rubrum dropped from 8 % to 0.15 % and 0.38 % respectively if lactic acid was mixed with propylene glycol in the ratios 3:10 or 2:3. The composition gave a minimal inhibitory effect at concentration 0.25% and 0.5 % respectively. These MIC-values should be compared with the considerably higher MIC-values of 40 %, which were obtained when propylene glycol at 20 % was combined with aluminium acetotartrate (SE 8802257-9). Optimum antibacterial effect was achieved in our own studies if the ratio between lactic acid and propylene glycol was 3:10, 3:7 or 2:3 dependent upon the type of microorganism. Also other concentration ratios gave stronger but not optimum effect. The antimicrobial effects were not proportionate to the degree of acidity (pH).

In a challenge test carried out according to the Apoteksbolaget's standard, where an inoculate of different microorganisms were added, a result was achieved closely comparable to disinfecting agents. Challenge testing was conducted with a varied concentration of bacteria 3 x 10⁵/ml, 5 x 10⁵/ml and 7 x 10⁵/ml. A complete and lasting inhibition (<1/ml) of three bacteria strains (Staphylococcus aureus, Escherichia coli and Pseudomonas) occurred already after 1 hour if an aqueous solution of merely 5 % lactic acid combined with 16 % propenylene glycol was used as test substance. When loaded with the mould fungus Aspergillus niger (2,2 x 10⁴/ml) and the yeast fungus Candida albicans (2,9 x 10⁵/ml) in the same test, the number of viable microorganisms/ml was <1 at a 48 hour reading. A logarithmic reduction of viable yeast fungi (Candida albicans) occurred after one hour from 2,9 x 10⁵/ml to 7,3 x 10¹/ml.

A determination of the minimum inhibitory concentration (MIC) was carried out for different compositions of acid/glycol on different microorganisms. Method description and the result are given below.

### Method description MIC-plate

The components included in the composition were weighed with decimal precision. A dilution series in sterile destilled water was made where the completed composition is dilution 1. Dilution 2 is 50 % of the composition, dilution 3 is 25 % etc. One thus gets a series of 100 - 50 - 25 - 12.5 - 6.3- 3.1 - 1.6 - 0.8 - 0.4 - 0.2 - 0.1 %. To said dilutions the same amount (volume) of Oxoids Isosensitest agar was added at 62.4 g medium per litre.

The Isosensitest agar is prepared and sterilized according to the maker's instructions. One then gets ready-to-use MIC-mixtures having 31.2 g Isosensitest medium per litre mixture and composition concentrations of 50 %, 25 % etc. down to 0.05 %. Furthermore a plate without the composition was prepared, called a blanc plate. From the different mixtures MIC-plates are moulded in sterile Petri dishes. From each agent a suspension was made corresponding to MacFarland 1. From this 0.2 ml was added to each MIC-plate and the blanc plate with a pipette. The MIC-plates were incubated in a thermostat having an optimum temperature for the agent. The MIC-value is the concentration of the composition on the MIC-plate where growth has stopped completely.
Example. Agent 1 grows in 0.1 %, 0.2 %, 0.4 %, On the 0.8 %-plate the growth is weak but visible. The MIC-value for agent 1 is then 1.6 %.

| MIC-determination of various compositions of acid/glycol pH in the various MIC plates | | | | | | | | | |
|---|---|---|---|---|---|---|---|---|---|
| Composition MIC-plate | 1 | 2 | 3 | 4 | 5 | 6 | 7 | 8 | 9 |
| 0 | 7 | 7 | 7 | 7 | 7 | 7 | 7 | 7 | 7 |
| 0.05 | 7 | 7 | 7 | 6.9 | 6.9 | 6.9 | 5.0 | 5.7 | 5.4 |
| 0.1 | 7 | 7 | 7 | 6.7 | 6.6 | 6.5 | 4.3 | 5.5 | 5.2 |
| 0.2 | 7 | 7 | 7 | 6.6 | 6.4 | 6.3 | 4.2 | 5.5 | 4.9 |
| 0.4 | 7 | 7 | 7 | 6.3 | 5.6 | 5.6 | 3.9 | 5.3 | 4.6 |
| 0.8 | 7 | 7 | 7 | 5.3 | 4.1 | 4.6 | 3.9 | 5.1 | 4.2 |
| 1.6 | 7 | 7 | 7 | 4.8 | 4.1 | 4.4 | 4.0 | 4.6 | 4.1 |
| 3.1 | 7 | 7 | 7 | 4.2 | 3.8 | 4.0 | 4.0 | 4.3 | 3.6 |
| 6.3 | 7 | 7 | 7 | 3.8 | 3.5 | 3.6 | 3.9 | 3.6 | 3.3 |
| 12.5 | 7 | 7 | 7 | 3.5 | 3.2 | 3.3 | 3.6 | 3.1 | 2.6 |
| 25 | 7 | 7 | 7 | 3.2 | 3.6 | 3.1 | 3.5 | 3.0 | 2.1 |
| 50 | 7 | 7 | 7 | 3.1 | 2.7 | 2.9 | 3.4 | 2.7 | 2.0 |

| MIC-value for various agents | | | | | | | | | |
|---|---|---|---|---|---|---|---|---|---|
| Composition Agent | 1 | 2 | 3 | 4 | 5 | 6 | 7 | 8 | 9 |
| A1 | 12.5 | 12.5 | 6.3 | 12.5 | 12.5 | 6.3 | 0.4 | 12.5 | 12.5 |
| A2 | 12.5 | 12.5 | 6.3 | 12.5 | 12.5 | 6.3 | 0.2 | 12.5 | 12.5 |
| B1 | 50 | 25 | 25 | 3.1 | 3.1 | 1.6 | 25 | 25 | 25 |
| B2 | 50 | 25 | 25 | 3.1 | 1.5 | 3.1 | 25 | 25 | 50 |
| C1 | 6.3 | 6.3 | 0.8 | 1.6 | 0.8 | 0.8 | <0.05 | 0.4 | 0.4 |
| C2 | 6.3 | 6.3 | 0.8 | 1.6 | 0.8 | 0.8 | <0.05 | 0.4 | 0.4 |
| D1 | 12.5 | 12.6 | 6.3 | 12.5 | 12.5 | 6.3 | 0.4 | 12.5 | 25 |
| D2 | 12.5 | 12.6 | 6.3 | 12.5 | 12.5 | 6.3 | 0.4 | 12.5 | 25 |
| E1 | 25 | 25 | 12.5 | 3.1 | 1.6 | 1.6 | 0.1 | 1.6 | 0.4 |
| E2 | 25.5 | 25 | 12.5 | 3.1 | 1.6 | 1.6 | 0.1 | 1.6 | 0.4 |
| F1 | 12.5 | 12.5 | 12.5 | 1.6 | 1.6 | 1.6 | 1.6 | 3.1 | 3.1 |
| F2 | 12.5 | 12.5 | 12.5 | 1.6 | 1.6 | 1.6 | 1.6 | 3.1 | 3.1 |
| G1 | 12.5 | 3.1 | 1.6 | 0.8 | 0.4 | 0.4 | <0.05 | 0.2 | 0.2 |
| G2 | 6.3 | 1.6 | 3.1 | 0.8 | 0.4 | 0.4 | <0.05 | 0.2 | 0.2 |

| | | |
|---|---|---|
| A1 and A2 | Candica albicans | Clinical isolate |
| B1 | Malassezia furfur | Pityrosporum ovale |
| B2 | Pityrosporum orbiculare | Pityrosporum ovale |
| C1 and C2 | Trichophyton rubrum | Clinical isolate |
| D1 and D2 | Aspergillus niger | Clinical isolate |
| E1 and E2 | Staphylococcus aureus | Clinical isolate |
| F1 and F2 | Streptococcus pyogenes | Clinical isolate |
| G1 and G2 | Pseudomonas aeruginosa | Clinical isolate |

| COMPOSITION | | |
|---|---|---|
| 1 | Propylene glycol | 100 % |
| 2 | Butylene glycol | 100 % |
| 3 | Hexylene glycol | 100 % |
| 4 | Lactic acid/propylene glycol | 30/70 % |
| 5 | Lactic acid/butylene glycol | 30/70 % |
| 6 | Lactic acid/hexylene glycol | 30/70 % |
| 7 | Acetic acid/propylene glycol | 30/70 % |
| 8 | Citric acid/propylene glycol | 30/70 % |
| 9 | Tartaric acid/propylene glycol | 30/70 % |
| Hexylene glycol = 2-methyl-2,4-pentane diol | | |
| Butylene glycol = 1,3-butane diol | | |
| Propylene glycol = 1,2-propane diol | | |

### Cosmetic and medical cleansing agents

The composition which is used according to the invention cleanses the skin due to the hygroscopic and antimicrobial properties and also because of the prominent peeling properties.

The composition according to the invention may also be used for preparing an agent having effect against such microorganisms which attack the skin and diseases caused by said microorganisms. Foremost, the composition according to the invention is effective against Candida, Pityrosporum, Trichophyton, Microsporum, mould fungi especially Aspergillus, Staphylococcus, Pseudomonas, and viruses, especially herpes virus, hepatitis virus, wart virus and HIV-virus.

Examples of diseases where these antimicrobial compositions show effectiveness are eczema, in particular seborrhoeic eczema and atopic eczema, as well as herpes and aphtha.

Preferred cosmetic and pharmaceutical areas of use are disinfection of skin, skin cleansing, sore and wound cleansing, as shampoos, soap, shower gels, softening preparations as well as peeling agents having antimicrobial effect, as skin preparations having antimicrobial effect intended to treat insect bites, as sun protection in combination with sunprotection agents or "after sun lotions". The composition may also be included as an additive in other cleansers and disinfecting agents to strengthen and widen the antimicrobial effects.

A composition for cosmetic or medical use according to the invention contains preferably 0.12-40 percent in weight low molecular carboxylic acid and 0.38-91 percent by weight diol.

Furthermore usual additives and vehicles used in cosmetics and medicine may also be included such as, for example, water, C₂-C₈-alcohols, polyalkylene glycols, oils with or without emulsifying agents, surfactants, antimycotics, antiviral agents, antibacterial agents, sulphur and sulphur compounds, glucocorticoids, gels and enzymes.

Urea may also be included in an amount of 1-20 percent by weight and thus thereby strengthen the penetration of the active substances or the effect of added enzyme.

A product containing the above defined carboxylic acids and C₃-C₁₀-diols passes the durability (shelf life) demands which are set by the authorities concerned and thus does not need the addition of preservatives. This means, for example, that use of a shampoo according to the invention fulfills the recommendations of the Swedish Society for the Conservation of Nature (Svenska Naturskyddsföreningen) for "good environmental choice". By means of the combination of carboxylic acid-diol, antimicrobial effect may be achieved with low concentrations of the diols included, which lessens the risk of side effects. This is especially favourable when the diol is used in dermatological vehicles. A combination of carboxylic acid and a diol gives a stable, homogeneous and lasting mixture, which is almost odour-free and also may be used as an aerosol. The components form stable solutions with water, ethyl alcohol and polyethylene glycols. Solutions containing propylene glycol, lactic acid, polyethylene glycol, urea and glycerol have been durability tested (shelf life tested) and have shown themselves to be durable for 3 years.

### Surface treatment

The composition according to the invention, preferably in the form of a mixture of diol and low molecular carboxylic acid, may be used for treatment, cleansing and disinfecting different surfaces, mainly biological material, such as leather, skin, wood, and also plastic, metal and ceramic materials.

Cleaning agents according to the invention differ from previously known cleaning agents and disinfecting agents containing, for example, ethyl alcohol and isopropanol, because they are both emollient and non-desiccating. Small skin cracks due to dryness of the skin often appear after cleansing with other disinfecting agents. Cleansing agents according to the invention have not caused skin cracks or drying out of the skin in the tests carried out. If the composition is used for the skin the effect works in two ways. Firstly, the infected horny layer (keratin) is removed in a mechanical way because both the components included have peeling-effects, which reduce the antigen. This reduced antigen may subsequently be killed by the antimicrobial effect of the composition.

Since the composition according to the invention is not volatile as ethyl alcohol it evaporates more slowly from the surface and therefor remains on and keeps its disinfecting effect for a longer time. From the fire-risk point of view the composition has also an advantage since it is comparatively less ignitible. Due to slow evaporation there are no obvious coiling effects compared to ethyl alcohol. The advantages are thus many in practical use of the invention if it is used as a cleaner for table surfaces, leather surfaces and plastic coated surfaces, e.g. dental surgery-chairs.

After cleaning leather and plastic an increased shine and surface finish has also been observed. Slight damage in the material has also appeared to be less obvious after treatment with the agent according to the invention. If the composition is used for wiping lacquered wood, certain types of lacquer may temporarily be dissolved on the surface. Thereafter it has been observed that previous damage in the lacquer is less obvious or invisible. These changes have been permanent. The product according to the invention may, thus, be used as an environmentally friendly and kind-to-the-skin cleansing agent and renovating agent for lacquered surfaces.

Since the composition may form an aerosol it may easily be sprayed over large areas for quicker application. Spraying may also be used for disinfection and cleansing of wrapping material, machine components, etc. or for preventing bacteria and mould attack on certain types of foodstuffs, e.g. bread, fruit, onions, as well as flower-bulbs, tobacco and animal fodder.

Another area of use for the composition according to the invention is to dissolve bacteria deposits on different surfaces or in cables or pipes.

For surface treatment of metals the composition may also be regarded as having rust dissolving effect in the light of what is previously known. Therefor the composition is suitable as an antimicrobial effective additive in anticorrosion agents.

As an additive in lubricants and cooling mediums, for example, lubricating oil and cutting fluids, the antimicrobial effect of the composition may be advantageously used so that other agents need not be added. By adding a buffer a suitable pH may be obtained as required.

The spraying of wood directly after sawing to inhibit the growth of surface-mould and to prevent inhalation fever in saw mill workers, is another area of use for the product according to the invention.

In one test surface-mould was inhibited on sawn up wood for 6 months by means of spraying just once with a mixture of lactic acid and propylene glycol. On the parts of the wood where the treatment did not take place, mould formation started already after 14 days.

### Impregnating

The composition according to the invention has shown itself to penetrate and be absorbed well by various materials. Treatment for preventing growth of microorganisms in deeper lying structures may be carried out by impregnating. Different materials suited for impregnating with the composition are leather; textiles, e.g. nappies, occlusive bandages, incontinence aids, tampongs, sanitary towels, dressings, plasters; clothes, innersoles for shoes; wood and paper products, e.g. refresher napkins, disposable towels, waste paper, etc.

The invention includes also the possibility of chemically bonding the impregnating agent according to the invention to, for example, free carboxylic acid groups in different material or to polymers absorbed or adsorbed to such material, for example, textiles, wood or paper material. Examples of such absorbed or adsorbed polymer are alginic acid or an alginate. Treatment according to this method then gives an antimicrobial impregnation which bonds more quickly through alginic acid to textiles and wood fibers. Examples of the area of use for this antimicrobial impregnation are tampongs, dressings for wounds and sores, nappies, incontinence aids and innersoles.

A gel of alginic acids or alginate to which the composition according to the invention is bonded may separately also serve as a skin and wound-care treatment without being absorbed by the textile material.

The composition may also be mixed with paints where oil is included or mixed in water-based acrylic paints to prevent mould and bacterial growth, instead of toxic anti-mould agents.

One problem when impregnating wood is that the combination according to the invention is leached by water. By adding an oil, which in itself is water repellent, this problem may be reduced to some extent. An emulsifying agent then needs to be added.

The hygroscopic effects, if used correctly, may also be an advantage since they can prevent crack-formation in wood. In the treatment of wood the impregnation with polyethylene glycols is an established method to obtain swelling of woodfibers and counter-act drying out and crack formation in the wood. Polyethylene glycols have, i.a. been used to preserve the Royal Ship Wasa. One problem which may arise with long-term treatment with polyethylene glycols is mould-formation, since polyethylene glycols do not have sufficient antimicrobial effect. Since the composition according to the invention is mixable with polyethylene glycols this new combination can prevent growth of microorganisms.

In the production of paper the risk of mould-formation is great. This is especially true of waste-paper. A further possibility of making use of the invention is, for example, to add the composition to the process water, or feed it in spray form to prevent mould-formation. Since the composition is water soluble it is easily added even to warm or cool water-systems, etc.

Summarizing, the combination of C₃-C₁₀-diols and the above defined carboxylic acids is of interest since it has strong and wide antimicrobial effects, while being kind to the skin and to different materials, without having negative environmental effects.

To prepare the cosmetic and pharmaceutical compositions for use according to the invention, the ingredients included were mixed according to generally accepted methods.

Examples of suitable preparations, which, however, shall not limit the scope, for use according to the invention are:-

### Example 1

| Shampoo | |
|---|---|
| Lactic acid | 3 g |
| Diol | 10 g |
| Glycerol | 5 g |
| Urea | 4 g |
| Polyethylene glycol | 5 g |
| Hydroxyethyl cellulose | 0.5 g |
| Surfactants | 30 g |
| Aqua pura to | 100 g |

### Example 2

| Soap | |
|---|---|
| Lactic acid | 3 - 5 g |
| Diol | 10 - 15 g |
| Glycerol | 5 - 10 g |
| Polyethylene glycol | 5 - 10 g |
| Hydroxyethyl cellulose (gel) | 0.5 g |
| Surfactants | 50 g |
| Aqua pura to | 100 g |

### Example 3

| Hand cleaning agent | | |
|---|---|---|
| a) | Lactic acid | 5 g |
| | Propylene glycol | 5 g |
| | Hexylene glycol | 10 g |
| | Glycerol | 10 g |
| | Olive oil | 20 g |
| | Surfactants | 40 g |
| | Emulsifying agent + water to | 100 g |
| | | |
| b) | Lactic acid | 5 g |
| | Propylene glycol | 5 g |
| | Hexylene glycol | 10 g |
| | Glycerol | 10 g |
| | Rape seed oil | 20 g |
| | Surfactants | 40 g |
| | Emulsifying agent + water to | 100 g |

### Example 4

| sore-cleansing agent | | |
|---|---|---|
| a) | Lactic acid | 2 g |
| | Propylene glycol | 15 g |
| | Aqua pura to | 100 g |
| | | |
| b) | Lactic acid | 2 g |
| | Hexylene glycol | 15 g |
| | Aqua pura to | 100 g |
| | | |
| c) | Lactic acid | 4 g |
| | Propylene glycol | 40 g |
| | Aqua pura to | 100 g |

Aqua pura may in examples 4a), 4b) and 4c) be replaced by diluted spirits or physiological saline. Gels, urea and enzymes may also be included.

### Example 5

| Skin preparation having antimicrobial effect | | |
|---|---|---|
| a) | Lactic acid | 6 - 18 g |
| | Propylene glycol | 10 - 30 g |
| | Hexylene glycol | 10 - 30 g |
| | Oil/water-emulsion to (or aqua pura) | 100 g |
| | | |
| b) | Lactic acid | 6 - 18 g |
| | Propylene glycol | 10 - 30 g |
| | Hexylene glycol | 10 - 30 g |
| | Urea | 4 - 15 g |
| | Glycerol | 10 g |
| | Oil/water-emulsion to (or aqua pura) | 100 g |
| c) | Lactic acid | 3 g |
| | Hexylene glycol | 10 g |
| | Glycerol | 10 g |
| | Oil/water-emulsion to (or aqua pura) | 100 g |
| | | |
| d) | Citric acid | 3 g |
| | Hexylene glycol | 10 g |
| | Glycerol | 10 g |
| | Oil/water-emulsion to (or aqua pura) | 100 g |

### Example 6

| Mould inhibiting agent and impregnating agent for wood | | |
|---|---|---|
| a) | Acetic acid | 20 - 30 g |
| | Propylene glycol to | 100 g |
| | | |
| b) | Lactic acid | 20 - 30 g |
| | Propylene glycol to | 100 g |
| | | |
| c) | Acetic acid | 20 - 30 g |
| | Hexylene glycol to | 100 g |
| | | |
| d) | Lactic acid | 20 - 30 g |
| | Hexylene glycol to | 100 g |
| | | |
| e) | Acetic acid | 10 g |
| | Propylene glycol | 30 g |
| | Polyethylene glycol to | 100 g |
| | | |
| f) | Acetic acid | 10 g |
| | Propylene glycol | 30 g |
| | Linseed oil to | 100 g |

### Example 7

| Surface improving agent for lacquered surfaces | |
|---|---|
| Lactic acid | 10 g |
| Propylene glycol to | 100 g |

### Example 8

| Surface improving anticeptic agent for leather and platic | |
|---|---|
| Lactic acid | 10 g |
| Propylene glycol | 40 g |
| Hexylene glycol | 30 g |
| Glycerol | 10 g |
| Aqua pura to | 100 g |

### Example 9

| Colouring agent having microbial effect | | |
|---|---|---|
| a) | Acetic acid | 3 - 5 g |
| | Hexylene glycol | 10 - 15 g |
| | Additional additives to | 100 g |
| | | |
| b) | Acetic acid | 3 - 5 g |
| | Propylene glycol | 10 - 15 g |
| | Additional additives to | 100 g |

### Example 10

| Disinfecting agent for surfaces (ex. wood, plastic, leather, skin) | | |
|---|---|---|
| a) | Acetic acid | 15 g |
| | Hexylene glycol | 70 g |
| | Aqua pura to | 100 g |
| b) | Lactic acid | 15 g |
| | Propylene glycol | 20 g |
| | Hexylene glycol | 50 g |
| | Aqua pura to | 100 g |
| c) | Lactic acid | 5 - 10 g |
| | Propylene glycol | 5 - 10 g |
| | Hexylene glycol | 10 - 20 g |
| | Aqua pura to | 100 g |

Instead of aqua pura in c) ethyl alcohol or isopropanol may be used. Only c) may be used as a skin disinfecting agent.

### Example 11

| Impregnation agent for textiles or refreshing napkins | | |
|---|---|---|
| a) | Lactic acid | 3 - 5 g |
| | Hexylene glycol | 10 - 15 g |
| | Aqua pura to | 100 g |
| | | |
| b) | Citric acid | 3 - 5 g |
| | Hexylene glycol | 10 - 15 g |
| | Aqua pura to | 100 g |
| | | |
| c) | Acetic acid | 3 - 5 g |
| | Hexylene glycol | 10 - 15 g |
| | Aqua pura to | 100 g |

The hexylene glycol in example 11 may be replaced by the same amount of propylene glycol and Aqua pura may be exchanged for physiological saline.

## Claims

1. A composition having hygroscopic, and antimicrobial effect comprising saturated and unsaturated, straight and branched aliphatic mono-, di- and poly-carboxylic acids having up to 10 carbon atoms and hydroxycarboxylic acids having up to 8 carbon atoms selected from formic acid, acetic acid, propionic acid, butyric acid, valeric acid, caproic acid, caprylic acid, capric acid, sorbic acid, oxalic acid, malonic acid, fumaric acid, succinic acid, glutaric acid, adipic acid, pimelic acid, oxalacetic acid, glycolic acid, citric acid, lactic acid, glucuronic acid, glyceric acid, malic acid, tartaric acid, tartronic acid, hydroxybutyric acid, hydroxypropionic acid and the salts thereof as well as C₃-C₁₀-diols selected from propylene glycol, butylene glycol, pentane diol, hexylene glycol, heptanediol, octanediol, nonanediol and decanediol, said composition comprising 23-60 percent by weight carboxylic acid and 40-77 percent by weight C₃-C₁₀-diol.

2. A composition according to claim 1, comprising 23-40 percent by weight of carboxylic acid and 60-77 percent by weight of C₃-C₁₀-diol.

3. A composition according to any of claims 1 to 2, wherein the carboxylic acids are selected from acetic acid, citric acid, tartaric acid, and lactic acid.

4. A composition according to any of the previous claims, wherein the carboxylic acid is selected from acetic acid, citric acid, tartaric acid, lactic acid and the diol selected from propylene glycol, butylene glycol, pentanediol and hexylene glycol.

5. A composition according to any of the previous claims wherein carboxylic acid to diol have a ratio to one another in the proportions of 3:10, 3:7 and 2:3.

6. A composition according to any of the previous claims further comprising additives and vehicles, such as water, C₁-C₈-alcohols, polymers, polyalkylene glycols, preferably polyethylene glycol, oils, with and without emulsifiers, surfactants, antimycotic agents, antiviral agents, antibacterial agents, sulphur and sulphur compounds, glucocorticoids, gels, enzymes.

7. A composition according to any of claims 1 to 6 comprising the diol in a dermatological vehicle.

8. Non-therapeutic use of a composition having hygroscopic, keratolytic and antimicrobial effect comprising, as active ingredient, saturated and unsaturated, straight and branched aliphatic mono-, di- and poly-carboxylic acids having up to 10 carbon atoms and hydroxycarboxylic acids having up to 8 carbon atoms selected from formic acid, acetic acid, propionic acid, butyric acid, valeric acid, caproic acid, caprylic acid, capric acid, sorbic acid, oxalic acid, malonic acid, fumaric acid, succinic acid, glutaric acid, adipic acid, pimelic acid, oxalacetic acid, glycolic acid, citric acid, lactic acid, glucuronic acid, glyceric acid, malic acid, tartaric acid, tartronic acid, hydroxybutyric acid, hydroxypropionic acid and the salts thereof as well as C₃-C₁₀-diols selected from propylene glycol, butylene glycol, pentane diol, hexylene glycol, heptanediol, octanediol, nonanediol and decanediol, said composition comprising 0.1-60 percent by weight carboxylic acid and 0.15-91 percent by we ight C₃-C₁₀-diol in anti-microbial cleaning, disinfecting, antimicrobial treatment, non-therapeutical surface treatment, impregnation or for the production of antimicrobial effective biologically decomposable material.

9. Non-therapeutic use according to claim 8 of a composition as defined above wherein the carboxylic acids are included in an amount of 9-60 percent by weight and the C₃-C₁₀-diols in an amount of 40-91 percent by weight.

10. Non-therapeutic use according to any of claims 8 to 9 of a composition as defined above wherein the carboxylic acids are included in an amount of 23-60 percent by weight and the C₃-C₁₀-diols in an amount of 40-77 percent by weight.

11. Non-therapeutic use according to any of claims 8 to 10 of a composition as defined above wherein the carboxylic acids are included in an amount of 9-40 percent by weight and the C₃-C₁₀-diols in an amount of 60-91 percent by weight.

12. Non-therapeutic use according to any of claims 8 to 11 of a composition as defined above wherein the carboxylic acids are included in an amount of 23-40 percent by weight and the C₃-C₁₀-diols in an amount of 60-77 percent by weight.

13. Non-therapeutic use according to any of claims 8 to 12 of a composition wherein the carboxylic acid is included in a ratio to diol in 9-40%, or preferably wherein carboxylic acid to diol have a ratio to one another in the proportions of 3:10, 3:7, and 2:3.

14. Non-therapeutic use according to any of claims 8 to 13, wherein the carboxylic acids are selected from acetic acid, citric acid, tartaric acid, and lactic acid.

15. Non-therapeutic use according to any of claims 8 to 14, wherein the carboxylic acid is selected from acetic acid, citric acid, tartaric acid, lactic acid and the diol selected from propylene glycol, butylene glycol, pentanediol and hexylene glycol.

16. Non-therapeutic use according to any of claims 8-15 in antimicrobial cleaning, disinfecting, surface treatment and impregnation of materials such as wood, leather, plastics, metal, ceramic materials, foodstuffs, paper and textiles, of a composition further comprising additives and vehicles such as water, C₁-C₈-alcohols, polymers, polyalkylene glycols, preferably polyethylene glycol, oils with or without emulsifiers, surfactants, antimycotic agents, antiviral agents, antibacterial agents, sulphur and sulphur compounds, urea in an amount of 1-20 percent by weight, gels and enzymes.

17. Non-therapeutic use according to any of claims 8 to 16 of the diol of the composition in a dermatological vehicle.

18. Non-therapeutic use according to any of claims 8 to 17 of a composition as defined above for the treatment of surfaces contaminated with yeast fungi in particular Candida albicans, Pityrosporum ovale/orbiculare, dermatophytes, in particular Trichophyton, Microsporum, mould fungi, in particular Aspergillus, bacteria, in particular Streptococcus pyogenes, Staphylococcus aureus, Pseudomonas aeruginosa, Escherichia coli and viruses, especially herpes virus, hepatitis virus, wart virus and HIV-virus,

19. Non-therapeutic use according to claim 8 of a composition as defined above as an additive in a composition wherein the carboxylic acid is included in an amount of 0.12-40 percent by weight and the diol in an amount of 0.38 -91 percent by weight.

20. Use of a composition having hygroscopic, keratolytic and antimicrobial effect comprising, as active ingredient, saturated and unsaturated, straight and branched aliphatic mono-, di- and poly-carboxylic acids having up to 10 carbon atoms and hydroxycarboxylic acids having up to 8 carbon atoms selected from formic acid, acetic acid, propionic acid, butyric acid, valeric acid, caproic acid, caprylic acid, capricacid, sorbic acid, oxalic acid, malonic acid, fumaric acid, succinic acid, glutaric acid, adipic acid, pimelic acid, oxalacetic acid, glycolic acid, citric acid, lactic acid, glucuronic acid, glyceric acid, malic acid, tartaric acid, tartronic acid, hydroxybutyric acid, hydroxypropionic acid and the salts thereof as well as C₃-C₁₀-diols selected from propylene glycol, butylene glycol, pentane diol, hexylene glycol, heptane diol, octanediol, nonanediol and decanediol, said composition comprising 0.1 -60 percent by weight carboxylic acid and 0.15-91 percent by weight C₃-C₁₀-diol, for preparing an agent for combatting microorganisms which attack the skin and diseases caused by these, for example eczema, herpes or aphthae.

21. Use according to claim 20 of a composition as defined above wherein the carboxylic acids are included in an amount of 9-60 percent by weight and the C₃-C₁₀-diols in an amount of 40- 91 percent by weight.

22. Use according to any of claims 20 to 21 of a composition as defined above wherein the carboxylic acids are included in an amount of 23-60 percent by weight and the C₃-C₁₀-diols in an amount of 40-77 percent by weight.

23. Use according to any of claims 20 to 22 of a composition as defined above wherein the carboxylic acids are included in an amount of 9-40 percent by weight and the C₃-C₁₀-diols in an amount of 60-91 percent by weight.

24. Use according to any of claims 20 to 23 of a composition as defined above wherein the carboxylic acids are included in an amount of 23-40 percent by weight and the C₃-C₁₀-diols in an amount of 60-77 percent by weight.

25. Use according to any of claims 20 to 24 of a composition as defined above wherein the carboxylic acid is included in a ratio to diol in 9-40%, or preferably wherein carboxylic acid to diol have a ratio to one another in the proportions of 3:10, 3:7, and 2:3.

26. Use according to any of claims 20 to 25, wherein the carboxylic acids are selected from acetic acid, citric acid, tartaric acid, and lactic acid.

27. Use according to any of claims 20 to 26, wherein the carboxylic acid is selected from acetic acid, citric acid, tartaric acid, lactic acid and the diol selected from propylene glycol, butylene glycol, pentanediol and hexylene glycol.

28. Use according to any of claims 20-27 in combatting microorganisms which attack the skin and diseases caused by these, for example eczema, herpes or aphthae, of a composition further comprising additives and vehicles such as water, C₁-C₈-alcohols, polymers, polyalkylene glycols, preferably polyethylene glycol, oils with or without emulsifiers, surfactants, antimycotic agents, antiviral agents, antibacterial agents, sulphur and sulphur compounds, glucocorticoids, gels and enzymes.

29. Use according to any of claims 20 to 28 of the diol of the composition in a dermatological vehicle.

30. Use according to any of claims 20 to 29 of a composition as defined above for preparing an agent for the purpose of combatting yeast fungi, in particular Candida albicans, Pityrosporum ovale/orbiculare, dermatophytes, in particular Trichophyton, Microsporum, mould fungi, in particular Aspergillus, bacteria in particular Streptococcus pyrogenes, Staphylococcus aureus, Pseudomonas aeruginosa, Escherichia coli and viruses, especially herpes virus, hepatitis virus, wart virus and HIV-virus.

31. Use according to claim 20 of a composition as defined above as an additive in a therapeutic composition for preparing an agent wherein the carboxylic acid is included in an amount of 0.12-40 percent by weight and the diol in an amount of 0.38 -91 percent by weight.

## Patentansprüche

1. Zusammensetzung mit einer hygroskopischen und antimikrobiellen Wirkung, umfassend gesättigte und ungesättigte, geradkettige und verzweigte aliphatische Mono-, Di- und Polycarbonsäuren mit bis zu 10 Kohlenstoffatomen und Hydroxycarbonsäuren mit bis zu 8 Kohlenstoffatomen, ausgewählt aus Ameisensäure, Essigsäure, Propionsäure, Buttersäure, Valeriansäure, n-Capronsäure, n-Caprylsäure, n-Caprinsäure, Sorbinsäure, Oxalsäure, Malonsäure, Fumarsäure, Bernsteinsäure, Glutarsäure, Adipinsäure, Pimelinsäure, Oxalessigsäure, Glycolsäure, Citronensäure, Milchsäure, Glucuronsäure, Glycerinsäure, Äpfelsäure, Weinsäure, Tartronsäure, Hydroxybuttersäure, Hydroxypropionsäure und den Salzen davon, sowie C₃-C₁₀-Diole, ausgewählt aus Propylenglycol, Butylenglycol, Pentandiol, Hexylenglycol, Heptandiol, Octandiol, Nonandiol und Decandiol, wobei die Zusammensetzung 23-60 Gew.-% Carbonsäure und 40-77 Gew.-% C₃-C₁₀-Diol umfasst.

2. Zusammensetzung nach Anspruch 1, umfassend 23-40 Gew.-% Carbonsäure und 60-77 Gew.-% C₃-C₁₀-Diol.

3. Zusammensetzung nach einem der Ansprüche 1 bis 2, wobei die Carbonsäuren ausgewählt sind aus Essigsäure, Citronensäure, Weinsäure und Milchsäure.

4. Zusammensetzung nach einem der vorangehenden Ansprüche, wobei die Carbonsäure ausgewählt ist aus Essigsäure, Citronensäure, Weinsäure, Milchsäure und das Diol ausgewählt ist aus Propylenglycol, Butylenglycol, Pentandiol und Hexylenglycol.

5. Zusammensetzung nach einem der vorangehenden Ansprüche, wobei Carbonsäure zu Diol in einem Verhältnis zueinander in den Proportionen von 3:10, 3:7 und 2:3 stehen.

6. Zusammensetzung nach einem der vorangehenden Ansprüche, außerdem umfassend Additive und Vehikel, wie etwa Wasser, C₁-C₈-Alkohole, Polymere, Polyalkylenglykole, vorzugsweise Polyethylenglycol, Öle, mit und ohne Emulgatoren, oberflächenaktive Stoffe, antimykotische Mittel, Antivirusmittel, antibakterielle Mittel, Schwefel und Schwefelverbindungen, Glucocorticoide, Gele, Enzyme.

7. Zusammensetzung nach einem der Ansprüche 1 bis 6, umfassend das Diol in einem dermatologischen Vehikel.

8. Nichttherapeutische Verwendung einer Zusammensetzung mit einer hygroskopischen, keratolytischen und antimikrobiellen Wirkung, umfassend als Wirkstoff gesättigte und ungesättigte, geradkettige und verzweigte aliphatische Mono-, Di- und Polycarbonsäuren mit bis zu 10 Kohlenstoffatomen und Hydroxycarbonsäuren mit bis zu 8 Kohlenstoffatomen, ausgewählt aus Ameisensäure, Essigsäure, Propionsäure, Buttersäure, Valeriansäure, n-Capronsäure, n-Caprylsäure, n-Caprinsäure, Sorbinsäure, Oxalsäure, Malonsäure, Fumarsäure, Bemsteinsäure, Glutarsäure, Adipinsäure, Pimelinsäure, Oxalessigsäure, Glycolsäure, Citronensäure, Milchsäure, Glucuronsäure, Glycerinsäure, Äpfelsäure, Weinsäure, Tartronsäure, Hydroxybuttersäure, Hydroxypropionsäure und den Salzen davon, sowie C₃-C₁₀-Diole, ausgewählt aus Propylenglycol, Butylenglycol, Pentandiol, Hexylenglycol, Heptandiol, Octandiol, Nonandiol und Decandiol, wobei die Zusammensetzung 0,1-60 Gew.-% Carbonsäure und 0,15-91 Gew.-% C₃-C₁₀-Diol umfasst, bei einer antimikrobiellen Reinigung, Desinfektion, antimikrobiellen Behandlung, nichttherapeutischen Oberflächenbehandlung, Imprägnierung oder zur Herstellung von antimikrobiell wirksamem biologisch zersetzbarem Material.

9. Nichttherapeutische Verwendung nach Anspruch 8 von einer wie vorstehend definierten Zusammensetzung, wobei die Carbonsäuren in einer Menge von 9-60 Gew.-% und die C₃-C₁₀-Diole in einer Menge von 40-91 Gew.-% enthalten sind.

10. Nichttherapeutische Verwendung nach einem der Ansprüche 8 bis 9 von einer wie vorstehend definierten Zusammensetzung, wobei die Carbonsäuren in einer Menge von 23-60 Gew.-% und die C₃-C₁₀-Diole in einer Menge von 40-77 Gew.-% enthalten sind.

11. Nichttherapeutische Verwendung nach einem der Ansprüche 8 bis 10 von einer wie vorstehend definierten Zusammensetzung, wobei die Carbonsäuren in einer Menge von 9-40 Gew.-% und die C₃-C₁₀-Diole in einer Menge von 60-91 Gew.-% enthalten sind.

12. Nichttherapeutische Verwendung nach einem der Ansprüche 8 bis 11 von einer wie vorstehend definierten Zusammensetzung, wobei die Carbonsäuren in einer Menge von 23-40 Gew.-% und die C₃-C₁₀-Diole in einer Menge von 60-77 Gew.-% enthalten sind.

13. Nichttherapeutische Verwendung nach einem der Ansprüche 8 bis 12 von einer Zusammensetzung, wobei die Carbonsäure in einem Verhältnis zu Diol in 9-40 % enthalten ist, oder vorzugsweise wobei Carbonsäure zu Diol in einem Verhältnis zueinander in den Proportionen von 3:10, 3:7 und 2:3 stehen.

14. Nichttherapeutische Verwendung nach einem der Ansprüche 8 bis 13, wobei die Carbonsäuren ausgewählt sind aus Essigsäure, Citronensäure, Weinsäure und Milchsäure.

15. Nichttherapeutische Verwendung nach einem der Ansprüche 8 bis 14, wobei die Carbonsäure ausgewählt ist aus Essigsäure, Citronensäure, Weinsäure, Milchsäure und das Diol ausgewählt ist aus Propylenglycol, Butylenglycol, Pentandiol und Hexylenglycol.

16. Nichttherapeutische Verwendung nach einem der Ansprüche 8-15 bei einer antimikrobiellen Reinigung, Desinfektion, Oberflächenbehandlung und Imprägnierung von Materialien wie Holz, Leder, Kunststoffen, Metall, keramischen Materialien, Lebensmitteln, Papier und Textilien, von einer Zusammensetzung, die außerdem Additive und Vehikel wie Wasser, C₁-C₈-Alkohole, Polymere, Polyalkylenglycole, vorzugsweise Polyethylenglycol, Öle mit oder ohne Emulgatoren, oberflächenaktive Stoffe, antimykotische Mittel, Antivirusmittel, antibakterielle Mittel, Schwefel und Schwefelverbindungen, Harnstoff in einer Menge von 1-20 Gew.-%, Gele und Enzyme umfasst.

17. Nichttherapeutische Verwendung nach einem der Ansprüche 8 bis 16 des Diols der Zusammensetzung in einem dermatologischen Vehikel.

18. Nichttherapeutische Verwendung nach einem der Ansprüche 8 bis 17 von einer wie vorstehend definierten Zusammensetzung zur Behandlung von Oberflächen, die mit Hefepilzen, insbesondere Candida albicans, Pityrosporum ovale/orbiculare, Dermatophyten, insbesondere Trichophyton, Mikrosporum, Schimmelpilzen, insbesondere Aspergillus, Bakterien, insbesondere Streptococcus pyogenes, Staphylococcus aureus, Pseudomonas aeruginosa, Escherichia coli und Viren, insbesondere dem Herpesvirus, Hepatitisvirus, Warzenvirus und HIV-Virus kontaminiert sind.

19. Nichttherapeutische Verwendung nach Anspruch 8 von einer wie vorstehend definierten Zusammensetzung als Additiv in einer Zusammensetzung, wobei die Carbonsäure in einer Menge von 0,12-40 Gew.-% und das Diol in einer Menge von 0,38-91 Gew.-% enthalten ist.

20. Verwendung einer Zusammensetzung mit einer hygroskopischen, keratolytischen und antimikrobiellen Wirkung, umfassend als Wirkstoff gesättigte und ungesättigte, geradkettige und verzweigte aliphatische Mono-, Di- und Polycarbonsäuren mit bis zu 10 Kohlenstoffatomen und Hydroxycarbonsäuren mit bis zu 8 Kohlenstoffatomen, ausgewählt aus Ameisensäure, Essigsäure, Propionsäure, Buttersäure, Valeriansäure, n-Capronsäure, n-Caprylsäure, n-Caprinsäure, Sorbinsäure, Oxalsäure, Malonsäure, Fumarsäure, Bernsteinsäure, Glutarsäure, Adipinsäure, Pimelinsäure, Oxalessigsäure, Glycolsäure, Citronensäure, Milchsäure, Glucuronsäure, Glycerinsäure, Äpfelsäure, Weinsäure, Tartronsäure, Hydroxybuttersäure, Hydroxypropionsäure und den Salzen davon, sowie C₃-C₁₀-Diole, ausgewählt aus Propylenglycol, Butylenglycol, Pentandiol, Hexylenglycol, Heptandiol, Octandiol, Nonandiol und Decandiol, wobei die Zusammensetzung 0,1-60 Gew.-% Carbonsäure und 0,15-91 Gew.-% C₃-C₁₀-Diol umfasst, zum Herstellen eines Mittels zum Bekämpfen von Mikroorganismen, welche die Haut angreifen, und von diesen verursachten Krankheiten, z.B. Ekzemen, Herpes oder Aphthen.

21. Verwendung nach Anspruch 20 von einer wie vorstehend definierten Zusammensetzung, wobei die Carbonsäuren in einer Menge von 9-60 Gew.-% und die C₃-C₁₀-Diole in einer Menge von 40-91 Gew.-% enthalten sind.

22. Verwendung nach einem der Ansprüche 20 bis 21 von einer wie vorstehend definierten Zusammensetzung, wobei die Carbonsäuren in einer Menge von 23-60 Gew.-% und die C₃-C₁₀-Diole in einer Menge von 40-77 Gew.-% enthalten sind.

23. Verwendung nach einem der Ansprüche 20 bis 22 von einer wie vorstehend definierten Zusammensetzung, wobei die Carbonsäuren in einer Menge von 9-40 Gew.-% und die C₃-C₁₀-Diole in einer Menge von 60-91 Gew.-% enthalten sind.

24. Verwendung nach einem der Ansprüche 20 bis 23 von einer wie vorstehend definierten Zusammensetzung, wobei die Carbonsäuren in einer Menge von 23-40 Gew.-% und die C₃-C₁₀-Diole in einer Menge von 60-77 Gew.-% enthalten sind.

25. Verwendung nach einem der Ansprüche 20 bis 24 von einer wie vorstehend definierten Zusammensetzung, wobei die Carbonsäure in einem Verhältnis zu Diol in 9-40 % enthalten ist, oder vorzugsweise wobei Carbonsäure zu Diol in einem Verhältnis zueinander in den Proportionen von 3:10, 3:7 und 2:3 stehen.

26. Verwendung nach einem der Ansprüche 20 bis 25, wobei die Carbonsäuren ausgewählt sind aus Essigsäure, Citronensäure, Weinsäure und Milchsäure.

27. Verwendung nach einem der Ansprüche 20 bis 26, wobei die Carbonsäure ausgewählt ist aus Essigsäure, Citronensäure, Weinsäure, Milchsäure und das Diol ausgewählt ist aus Propylenglycol, Butylenglycol, Pentandiol und Hexylenglycol.

28. Verwendung nach einem der Ansprüche 20 bis 27 beim Bekämpfen von Mikroorganismen, welche die Haut angreifen, und von diesen verursachten Krankheiten, z.B. Ekzemen, Herpes oder Aphthen, von einer Zusammensetzung, die außerdem Additive und Vehikel wie Wasser, C₁-C₈-Alkohole, Polymere, Polyalkylenglycole, vorzugsweise Polyethylenglycol, Öle mit oder ohne Emulgatoren, oberflächenaktive Stoffe, antimykotische Mittel, Antivirusmittel, antibakterielle Mittel, Schwefel und Schwefelverbindungen, Glucocorticoide, Gele und Enzyme umfasst.

29. Verwendung nach einem der Ansprüche 20 bis 28 des Diols der Zusammensetzung in einem dermatologischen Vehikel.

30. Verwendung nach einem der Ansprüche 20 bis 29 von einer wie vorstehend definierten Zusammensetzung zum Herstellen eines Mittels zum Zweck der Bekämpfung von Hefepilzen, insbesondere Candida albicans, Pityrosporum ovale/orbiculare, Dermatophyten, insbesondere Trichophyton, Microsporum, Schimmelpilzen, insbesondere Aspergillus, Bakterien, insbesondere Streptococcus pyogenes, Staphylococcus aureus, Pseudomonas aeruginosa, Escherichia coli und Viren, insbesondere dem Herpesvirus, Hepatitisvirus, Warzenvirus und HIV-Virus.

31. Verwendung nach Anspruch 20 von einer wie vorstehend definierten Zusammensetzung als Additiv in einer therapeutischen Zusammensetzung zum Herstellen eines Mittels, wobei die Carbonsäure in einer Menge von 0,12-40 Gew.-% und das Diol in einer Menge von 0,38-91 Gew.-% enthalten ist.

## Revendications

1. Une composition ayant des effets hygroscopiques et antimicrobiens comprenant des acides mono-, di- et polycarboxyliques aliphatiques saturés et insaturés, linéaires et ramifiés, comportant jusqu'à 10 atomes de carbone, et des acides hydroxycarboxyliques composant jusqu'à 8 atomes de carbone, choisis parmi l'acide formique, l'acide acétique, l'acide butyrique, l'acide valérique, l'acide caproïque, l'acide capylique, l'acide caprique, l'acide sorbique, l'acide oxalique, l'acide malonique, l'acide fumarique, l'acide succinique, l'acide glutarique, l'acide adipique, l'acide pimélique, l'acide oxalacétique, l'acide glycolique, l'acide citrique, l'acide lactique, l'acide glucoronique, l'acide glycérique, l'acide malique, l'acide tartrique. l'acide tartronique, l'acide hydroxybutyrique, l'acide hydroxypropionique et les sels correspondants ainsi que des diols en C₃-C₁₀ choisis parmi le propylène glycol, le butylène glycol, le pentanediol, l'hexylène glycol. l'heptanediol, l'octanediol, le nonanediol et le décanediol, ladite composition comprenant 23-60% en poids d'acide carboxylique et 40-77% en poids de diol en C₃-C₁₀.

2. Une composition selon la revendication 1, comprenant 23-40% en poids d'acide carboxylique, et 60-77% en poids de diol en C₃-C₁₀.

3. Une composition selon une quelconque des revendications 1 ou 2, dans laquelle les acides carboxyliques sont choisis parmi l'acide acétique, l'acide citrique, l'acide tartrique et l'acide lactique.

4. Une composition selon une quelconque des revendications précédentes, dans laquelle l'acide carboxylique est choisi parmi l'acide acétique, l'acide citrique, l'acide tartrique, l'acide lactique, et le diol est choisi parmi le propylène glycol, le butylène glycol, le pentanediol et l'hexylène glycol.

5. Une composition selon une quelconque des revendications précédentes, dans laquelle le rapport de l'acide carboxylique au diol est dans les proportions de 3/10, 3/7 et 2/3.

6. Une composition selon une quelconque des revendications précédentes, comprenant en outre des additifs et des véhicules comme eau, alcools en C₁-C₈, polymères, polyalkylène glycol, de préférence polyéthylène glycol, des huiles, avec ou sans émulsifiants, agents tensioactifs, agents antimycosiques, agents antiviraux, agents antibactériens, soufre et dérivés de soufre, glucocorticoïdes, gels, enzymes.

7. Une composition selon une quelconque des revendications 1 à 6, comprenant le diol dans un véhicules dermatologique.

8. L'Utilisation non thérapeutique d'une composition ayant des effets hygroscopiques et antimicrobiens comprenant des acides mono-. di- et polycarboxyliques aliphatiques saturés et insaturés, linéaires et ramifiés, comportant jusqu'à 10 atomes de carbone, et des acides hydroxycarboxyliques et comportant jusqu'à 8 atomes de carbone, choisis parmi l'acide formique, l'acide acétique, l'acide butyrique, l'acide valérique, l'acide caproïque, l'acide capylique, l'acide caprique, l'acide sorbique, l'acide oxalique, l'acide malonique, l'acide fumarique, l'acide succinique, l'acide glutarique, l'acide adipique, l'acide pimélique, l'acide oxalacétique, l'acide glycolique, l'acide citrique, l'acide lactique, l'acide glucoronique, l'acide glycérique, l'acide malique, l'acide tartarique, l'acide tartronique, l'acide hydroxybutyrique, l'acide hydroxypropionique et les sels correspondants ainsi que des diols en C₃-C₁₀ choisis parmi le propylène glycol, le butylène glycol, le pentanediol, l'hexylène glycol, l'heptanediol, l'octanediol, le nonanediol et le décanediol, ladite composition comprenant 0,1 à 60% en poids d'acide carboxylique, 0,15-91% en poids de diols en C₃-C₁₀, pour l'assainissement antimicrobien, la désinfection, le traitement antimicrobien, le traitement de surface non thérapeutique, l'imprégnation ou pour la production de produits biologiquement dégradables efficaces comme antimicrobiens.

9. L'utilisation non thérapeutique selon la revendication 8 d'une composition telle que définie ci-dessus, dans laquelle les acides carboxyliques sont incorporés à raison de 9 à 60% en poids, et les diols en C₃-C₁₀, à raison de 40 à 91% en poids.

10. L'utilisation non thérapeutique selon une quelconque des revendications 8 ou 9 d'une composition telle que définie ci-dessus, dans laquelle les acides carboxyliques sont incorporés à raison de 23 à 60% en poids, et les diols en C₃-C₁₀, à raison de 40 à 77% en poids.

11. L'utilisation non thérapeutique selon une quelconque des revendications 8 à 10 d'une composition telle que définie ci-dessus, dans laquelle les acides carboxyliques sont incorporés à raison de 9 à 40% en poids, et des diols en C₃-C₁₀, à raison de 60 à 91 % en poids.

12. L'utilisation non thérapeutique selon une quelconque des revendications 8 à 11, d'une composition telle que définie ci-dessus dans laquelle les acides carboxyliques sont incorporés à raison de 23 à 40% en poids, et les diols en C₃-C₁₀, à raison de 60 à 77% en poids.

13. L'utilisation non thérapeutique selon une quelconque des revendications 8 à 12 d'une composition où l'acide carboxylique est incorporé selon un rapport aux diols de 9 à 40%, ou de préférence où le rapport de l'acide carboxylique au diol est de préférence de 3/10, 3/7 et 2/3.

14. L'utilisation non thérapeutique selon une quelconque des revendications 8 à 13, dans laquelle les acides carboxyliques sont choisis parmi les acides acétique, acide citrique, acide tartrique, et acide lactique.

15. L'utilisation non thérapeutique selon une quelconque des revendications 8 à 14, dans laquelle l'acide carboxylique est choisi parmi les acides acétique, acide citrique, acide tartrique, acide lactique, et le diol est choisi parmi propylène glycol, butylène glycol, pentanediol et hexylène glycol.

16. L'utilisation non thérapeutique selon une quelconque des revendications 8 à 15, pour l'assainissement antimicrobien, la désinfection, le traitement de surface et l'imprégnation de matières, telles que bois, cuir, matière plastique, métal céramique, produits alimentaires, papiers et textiles, d'une composition comprenant en outre des additifs et des véhicules comme l'eau, des alcools en C₁-C₈, des polymères, des polyalkylène glycols, de préférence polyéthylène glycol, des huiles, avec ou sans émulsifiants, agents tensioactifs, agents antimycosiques, agents antiviraux, agents bactériens, soufre et dérivés de soufre, urée à raison de I à 20% en poids, en gels et enzymes.

17. L'utilisation non thérapeutique selon une quelconque des revendications 8 à 16, du diol de la composition dans un véhicules dermatologique.

18. L'utilisation non thérapeutique selon une quelconque des revendications 8 à 17, d'une composition telle que défini ci-dessus, pour le traitement de surfaces contaminées par des champignons de levure. en particulier Candida albicans, Pityrosporum ovale/orbiculare, des dermatophytes, en particulier Trichophyton, Microsporum, des champignons de moisissure, en particulier Aspergillus, des bactéries, en particulier Streptococcus pyogenes, Staphylococcus aureus, Pseudomonas aeruginosa, Escherichia coli et des virus, en particulier le virus de l'herpes, le virus de l'hépatite, le virus des verrues et le virus du HIV.

19. L'utilisation non thérapeutique selon la revendication 8 d'une composition telle que définie ci-dessus, en tant qu'additif dans une composition où l'acide carboxylique est incorporé à raison de 0,12-40% en poids, et le diol à raison de 0.38-91% en poids.

20. L'utilisation d'une composition ayant des effets hygroscopiques, keratolitiques et antimicrobiens comprenant en tant qu'ingrédient actif, des acides mono-, di- et polycarboxyliques, saturés et insaturés, linéaires et ramifiés, comportant jusqu'à 10 atomes de carbone, et des acides hydroxycarboxyliques comportant jusqu'à 8 atomes de carbone, choisis parmi l'acide formique, l'acide acétique, l'acide butyrique, l'acide valérique, l'acide caproïque, l'acide capylique, l'acide caprique, l'acide sorbique, l'acide oxalique, l'acide malonique, l'acide fumarique, l'acide succinique, l'acide glutarique, l'acide adipique, l'acide pimélique, l'acide oxalacétique, l'acide glycolique, l'acide citrique, l'acide lactique, l'acide glucoronique, l'acide glycérique, l'acide malique, l'acide tartarique, l'acide tartronique, l'acide hydroxybutyrique, l'acide hydroxypropionique et les sels correspondants ainsi que des diols en C₃-C₁₀ choisis parmi propylène glycol, butylène glycol, pentanediol, hexylène glycol, heptanediol, octanediol, nonanediol et décanediol, ladite composition comprenant 0.1-60% en poids d'acide carboxylique, et 0.15-91% en poids de diol en C₃-C₁₀, pour la préparation d'un agent pour combattre les microorganismes qui s'attaquent à la peau, et pour le traitement de maladies provoquées par ceux-ci, par exemple eczéma, herpès ou aphtose.

21. L'utilisation selon la revendication 20 d'une composition telle que définie ci-dessus, dans laquelle les acides carboxyliques sont incorporés à raison de 9-60% en poids et le diol en C₃-C₁₀ à raison de 40-91% en poids.

22. L'utilisation selon une quelconque des revendications 20 à 21 d'une composition telle que définie ci-dessus dans laquelle les acides carboxyliques sont incorporés à raison de 23 à 60% en poids, et les diols en C₃-C₁₀ à raison de 40 à 77% en poids.

23. L'utilisation selon une quelconque des revendications 20 à 22 d'une composition telle que définie ci-dessus, dans laquelle les acides carboxyliques sont incorporés à raison de 9-40% en poids. et les diols en C₃-C₁₀ à raison de 60 à 91% en poids.

24. L'utilisation selon une quelconque des revendications 20 à 23 d'une composition telle que définie ci-dessus dans laquelle les acides carboxyliques sont incorporés à raison de 23-40% en poids. et les diols en C₃-C₁₀ à raison de 60 à 77% en poids.

25. L'utilisation selon une quelconque des revendications 20 à 24 d'une composition telle que définie ci-dessus dans laquelle l'acide carboxylique est incorporé selon un rapport au diol de 9-40%, ou de préférence où le rapport de l'acide carboxylique au diol se trouve dans les proportions de 3/10, 3/7, et de 2/3.

26. L'utilisation selon une quelconque des revendications 20 à 25 dans laquelle les acides carboxyliques sont choisis parmi l'acide acétique. l'acide citrique, l'acide tartrique et l'acide lactique.

27. L'utilisation selon une quelconque des revendications 20 à 26 dans laquelle l'acide carboxylique est choisi parmi l'acide acétique, l'acide citrique, l'acide tartrique, l'acide lactique, et le diol est choisi parmi le propylène glycol, le butylène glycol, le pentanediol et l'hexylène glycol.

28. L'utilisation selon une quelconque des revendications 20 à 27, pour combattre les microorganismes qui s'attaquent à la peau et les maladies provoquées par ceux-ci. par exemple eczéma et herpès ou aphtes, d'une composition comprenant en outre des additifs et véhicules tels que eau, alcool, en C₁-C₈, polymères, polyalkylène glycols, de préférence polyéthylène glycols, huiles avec ou sans émulsifiant, agents tensioactifs, agents antimycosiques, agents antiviraux, agents antibactériens, soufre et dérivés de soufre, glucocorticoïdes, gels et enzymes.

29. L'utilisation selon une quelconque des revendications 20 à 28 du diol de la composition dans un véhicules dermatologique.

30. L'utilisation selon une quelconque des revendications 20 à 29 d'une composition telle que définie ci-dessus, pour préparer un agent afin de combattre les champignons de levure, en particulier Candida albicans, Pityrosporum ovale/orbiculare, les dermatophytes, en particulier Trichophyton, Microsporum, les champignons de moisissure, en particulier Aspergillus, les bactéries, en particulier Streptococcus pyogenes, Staphylococcus aureus, Pseudomonas aeruginosa, Escherichia coli, et des virus, en particulier virus de l'herpès, virus de l'hépatite, virus des verrues et virus du HIV.

31. L'utilisation selon la revendication 20 d'une composition telle que définie ci-dessus, en tant qu'additif dans une composition thérapeutique pour préparer un agent dans lequel l'acide carboxylique est incorporé à raison de 0.12-40% en poids, et le diol à raison de 0.38-91% en poids.
